# EUROPEAN PATENT SPECIFICATION

(11) **EP 4 271 401 B1**
(45) Date of publication and mention of the grant of the patent: **20.05.2026**
(21) Application number: 22734775.4
(22) Date of filing: 03.01.2022
(51) Int. Cl.: A61K 38/00, C07K 1/00, C07K 1/04, C07K 1/06, C07K 14/605

(54) **SYNTHESIS OF TEDUGLUTIDE**
SYNTHESE VON TEDUGLUTINID
SYNTHÈSE DE TÉDUGLUTIDE

(30) Priority: 04.01.2021 IN 202141000174
(43) Date of publication of application: 08.11.2023
(73) Proprietor: Biocon Limited, Bangalore 560 100 Karnataka (IN)
(72) Inventor: GANGA RAMU, Vasanthakumar, Karnataka Bengaluru 560004 (IN); PATIL, Nitin, Karnataka Bangalore 560034 (IN); SUVARNA, Deepa Shankar, India Bengaluru 560 095 (IN)
(74) Representative: Michalski Hüttermann & Partner Patentanwälte mbB
(86) International application number: PCT/IB2022/050013
(87) International publication number: WO 2022/144860

(56) References cited:
- CN-A- 104 072 603
- CN-A- 104 072 605
- CN-A- 104 418 949
- US-A1- 2013 310 314
- ALBERICIO FERNANDO ET AL: "Solid-phase synthesis of peptides with C -terminal asparagine or glutamine : An effective, mild procedure based on N x -fluorenylmethyloxycarbonyl (Fmoc) protection and side-chain anchoring to a tris(alkoxy)benzylamide (PAL) handle*", INTERNATIONAL JOURNAL OF PEPTIDE AND PROTEIN RESEARCH., vol. 35, no. 3, 1 March 1990 (1990-03-01), DK, pages 284 - 286, XP093222333, ISSN: 0367-8377, DOI: 10.1111/j.1399-3011.1990.tb00950.x

## Description

### Related Application:

This application claims the benefit of priority of our Indian patent application IN 202141000174 filed on January 04, 2021.

### TECHNICAL FIELD

The present invention relates to the efficient solid-phase synthesis of Teduglutide represented by **Formula-I** (SEQ ID NO: 1).

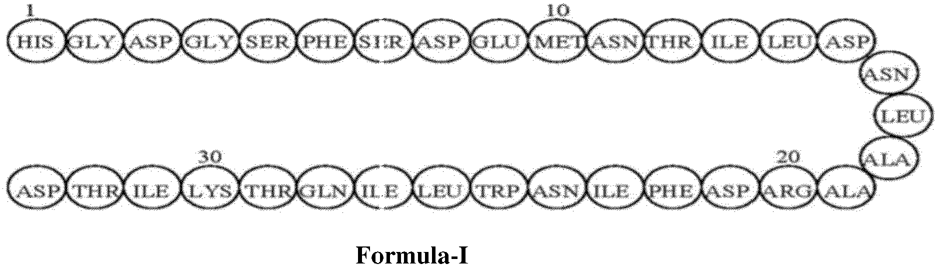

### BACKGROUND

GATTEX^{®} (Teduglutide) is a glucagon-like peptide-2 (GLP-2) analog indicated for the treatment of adults and pediatric patients 1 year of age and older with Short Bowel Syndrome (SBS) who are dependent on parenteral support.

The active ingredient in GATTEX (teduglutide) for injection is teduglutide, which is a 33 amino acid glucagon-like peptide-2 (GLP-2) analog manufactured using a strain of Escherichia coli modified by recombinant DNA technology.

The chemical composition of teduglutide is L-histidyl-L-glycyl-L-aspartyl-L-glycyl-L-seryl-L-phenylalanyl-L-seryl-L-aspartyl-L-glutamyl-L-methionyl-L-asparaginyl-L-threonyl-L-isoleucyl-L-leucyl-L-aspartyl-L-asparaginyl-L-leucyl-L-alanyl-L-alanyl-L-arginyl-L-aspartyl-L-phenylalanylL-isoleucyl-L-asparaginyl-L-tryptophanyl-L-leucyl-L-isoleucyl-L-glutaminyl-L-threonyl-L-lysyl-L-isoleucyl-Lthreonyl-L-aspartic acid.

Teduglutide has a molecular weight of 3752 Daltons. Teduglutide drug substance is a clear, colorless to light-straw-colored liquid.

US 5,789,379 B1 discloses Teduglutide and process for preparing it.

Many publications of prior art disclosures teach fragment phase synthesis of Teduglutide.

WO2012028602A1 discloses fragment phase synthesis of Teduglutide, by first synthesizing amino acid fragments at positions 1-4 and 5-33, and then coupling them to obtain Teduglutide.

CN104072605A discloses three fragment phase syntheses methods of Teduglutide. One is to first synthesize amino acid fragments at positions 1-9, 10-18, and 19-33, and then Combine each fragment to obtain Teduglutide; second, first synthesize 1-4, 5-12, 13-20, 21-33 amino acid fragments, and then couple each fragment to obtain Teduglutide; and third, first synthesize 1-4, 5-9, 10-18, 19-26, 27-33 amino acid fragments, and then coupling each fragment to obtain Teduglutide.

CN104418949A discloses a method for preparing Teduglutide, by first synthesizing amino acid fragments at positions 1-3 and 4-33, and then coupling them to obtain Teduglutide.

CN104817638 discloses synthesizing fragments 1-2, 3-4 and 5-33 and coupling said fragments together to obtain Teduglutide.

CN104072603 discloses the synthesis of Teduglutide by coupling a His residue with a fragment 2-33.

The synthesis methods described in the above patents are to synthesize the fragments first, and then connect the fragments to synthesize teduglutide. These methods require the synthesis of two or more polypeptide fragments to be connected. This approach needs fragments to be connected one by one on the solid support, then the full protection is cut off, and then the fragments are connected one by one. The biggest problem is that when fragments undergo fully protected cleavage, some side chain protecting groups may fall off and fragment impurities will be introduced during the subsequent liquid phase connection. At the same time, if the purity of fully protected fragments is low, it may be necessary to purify these fragments, which further increases the complexity of the process. Secondly, in the fragment condensation process, in order to ensure the complete reaction, it is usually necessary to feed the fragments in multiple amounts, resulting in a lot of waste of raw materials. Moreover, in the process of fragment ligation, racemization of amino acids is often unavoidable, which also brings great challenges to subsequent purification.

### Advantages of the present invention/specification:

The present invention is defined by the appended independent claims 1 and 12. Peptide synthesis can be accomplished by standard solid phase approach through Fmoc-/tBu strategy. However, there are multiple side reactions involved during the synthesis which impacts the yield and quality of the final product.

Disclosed herein is a novel synthetic approach of solid phase synthesis of peptides with C-terminal Aspartic acid by anchoring the side chain carboxylic group of aspartic acid to the solid support to avoid the formation of various impurities and thus resulting in higher yield and ease the purification process. One of the major impurities is the formation of diketopiperazine (DKP) impurity. DKP is the result of one of the side reactions in solid phase peptide synthesis which results in deletion sequences. Intermolecular cyclization occurs during deprotection of the Fmoc group from the N-terminal end of the dipeptide attached to the Wang resin.

The process provided herein is useful in solid phase synthesis of a wide class of peptides with C-terminal Aspartic acid. Prominent examples of this class of peptides are Arginylglycylaspartic acid (RGD) peptides which is found within many matrix proteins, including fibronectin, fibrinogen, vitronectin, osteopontin and the like. Eptifibatide is another example.

Present invention/specification remedies the above problem in the process for preparation of Teduglutide wherein the DKP impurity formation is minimized by anchoring of first amino acid, aspartic acid to the Wang resin through its side chain carboxylic group (Fmoc-Asp-OtBu) as against α-carboxylic group of Fmoc-Asp(OtBu)-OH, which is a standard procedure employed. When Fmoc-Asp-OtBu is used, a 7-memerberd ring has to be formed which is difficult whereas when Fmoc-Asp(OtBu)-OH is used, kinetically stable 6-membered diketopiperazine formation is observed which in turn leads to leaching of peptide from Wang resin and results with Thr-Asp Clipped impurity. Thus, present aspect of invention/specification provides for synthesis of Teduglutide wherein formation of diketopiperazine impurity is avoided.

### a) Role of anchoring the side chain carboxylic group of aspartic acid to the solid support: Diketopiperazine (DKP) impurity:

One of the common hurdles is formation of diketopiperazine (DKP) impurity. DKP is resultant of one of the side reactions in solid phase peptide synthesis which results in deletion sequences. The intermolecular cyclization occurs during deprotection of Fmoc group from the N-terminal end of the dipeptide attached to the Wang resin. In case of Teduglutide, as the first two amino acids are aspartic acid and threonine, the intermolecular cyclization results in leaching of peptide from the resin and the attachment of third amino acid in the sequence to the Wang resin thus leading to the formation Des Thr-Asp impurity.

### Mechanism for DKP formation:

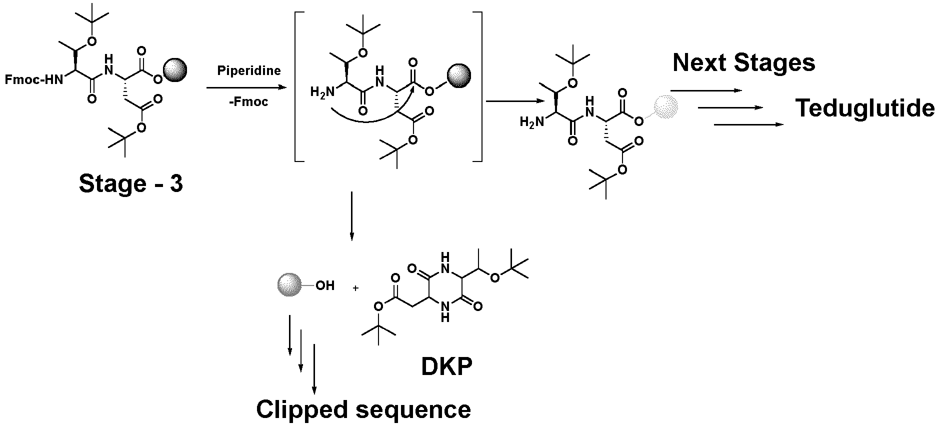

Present invention/specification remedies the above problem in the process for preparation of Teduglutide wherein the DKP impurity formation is minimized by anchoring of first amino acid, aspartic acid to the Wang resin through its side chain carboxylic group (Fmoc-Asp-OtBu) as against α-carboxylic group of Fmoc-Asp(OtBu)-OH, which is a standard procedure employed. When Fmoc-Asp-OtBu is used, a 7-memerberd ring has to be formed which is difficult whereas when Fmoc-Asp(OtBu)-OH is used, kinetically stable 6-membered diketopiperazine formation is observed which in turn leads to leaching of peptide from Wang resin and results with Thr-Asp Clipped impurity. Thus, present aspect of invention/ specification provides for synthesis of Teduglutide wherein formation of diketopiperazine impurity is avoided.

| **Raw material used for first loading** | | **Possible impurity** |
|---|---|---|
| Fmoc-Asp(O*^{t}*Bu)-OH | | 6 membered ring: Formation is easy |
| 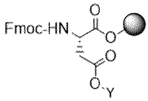 | [Y is O*^{t}*Bu] | |
| Fmoc-Asp-O*^{t}*Bu | | 7 membered ring: Formation is difficult |
| 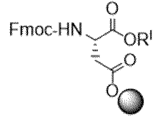 | [R¹ is O*^{t}*Bu] | |

### b) Role of free amino acids and antioxidant in TFA cocktail for cleaving the peptide from the resin:

Presence of Tryptophan and Methionine in the sequence leads to oxidation during cleaving and isolation of the peptide from the resin, this results in impurity formation, yield loss and addition of purification steps to remove these impurities rendering the process un-economical. One of the aspects of the present invention/specification remedies the above problem by incorporating corresponding free amino acids in the cleavage cocktail. The addition of free tryptophan and methionine amino acids found to be advantageous in minimizing oxidative impurities. The present invention/specification further involves the use of an antioxidant, selected from BHT and the like in the cleavage cocktail. These additional scavenges shares the oxidation load with desired peptide and in-turn minimizes oxidative impurities. This invention is particularly useful in peptides containing Tryptophan and Methionine in their sequence which can undergo oxidation during cleavage and isolation of the peptide. The non-limiting examples for this invention are Teduglutide, Glucagon and the like.

### Oxidative impurities of Methionine:

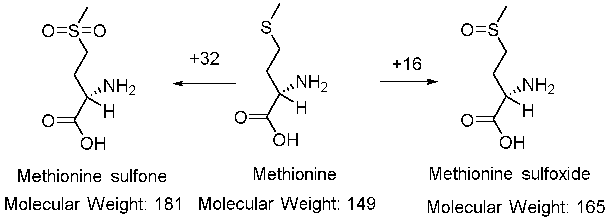

### Oxidative impurities of Tryptophan:

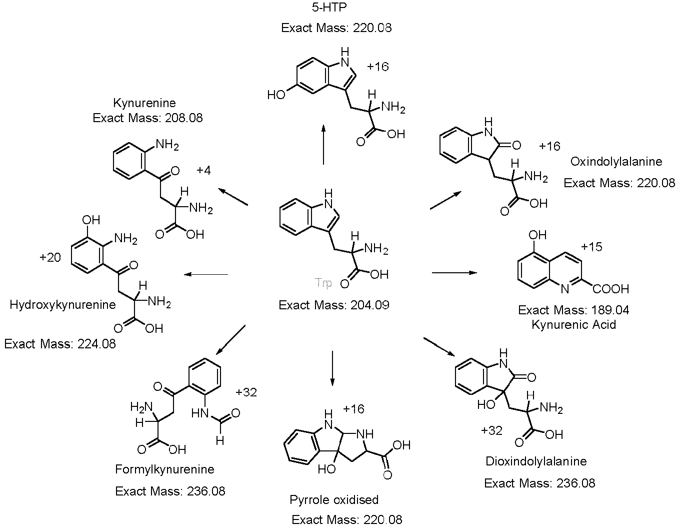

### c) Advantages of using Boc-His-OH:

Prior art methods use dual protection on Histidine like Fmoc-His(Trt)-OH, di-Boc groups, Boc-His(Trt)-OH, Boc-His(Bom)-OH and the like. Coupling of di-protected His is sluggish because of steric hindrance and it can lead to racemization. Further, di-protected amino acid derivative require additional purification either by chromatographic techniques or crystallization to separate the un-wanted τ-isomer and hence large-scale manufacturing is not cost effective. Whereas unprotected imidazole group of His can lead to extension of peptide through π-nitrogen of the imidazole ring and considerable racemization is possible if His is in middle of the sequence. Since, His is the last amino acid in Teduglutide sequence di-protection on His is not essential. This avoids use of expensive di-protected amino acid. Further, the presently used coupling conditions avoids racemization significantly. Hence usage of Histidine amino acid with free side chain would not create problems during teduglutide manufacturing process.

### d) Advantages of using COMU/HCTU/T3P as coupling agent using γ-valerolactone as solvent:

In peptide synthesis, the choice of coupling reagents varies based on the sequence and incoming amino acids. COMU/HCTU/T3P are more advanced coupling reagents used during coupling. COMU in particular, is more efficient reagent, it reduces racemization and it has got less hazardous safety profile compared to HATU and HBTU. On the other side, HCTU and T3P are efficient and cost effective. It is found that in the present invention/specification when COMU/HCTU/T3P used as coupling agent in combination with γ-valerolactone as solvent the reagents were stable, and these conditions suppressed the racemization. The above combination is also environmentally friendly.

### e) Advantages of use of ACN and MIBK during the isolation of peptide after global cleavage:

After the global cleavage, the TFA cocktail containing the peptide was concentrated and precipitation of crude peptide was carried out using ethers such as MTBE, diethyl ether and diisopropyl ether as antisolvent. If we use ethers as antisolvents for the precipitation of the peptide, assay of crude peptide drops to 20-25 %. This is due to the precipitation of side chain protecting group and scavenger adducts along with desired peptide. The assay of the crude peptide is increased by precipitating crude peptide with ACN or MIBK. The adducts will remain in mother liquor and gets removed during filtration. In addition, Teduglutide is prone for oxidation because of the presence of amino acids like methionine, tryptophan, and Histidine in its sequence. It is known ethers contains traces of peroxides which leads to increase in oxidised impurities. Usage of acetonitrile contributed significantly to reduce oxidised impurities. The purity and assay comparison of crude peptide are tabulated as follows.

| **Solvent used for isolation** | **Purity (%)** | **Assay (%)** |
|---|---|---|
| MTBE | 47.70 | 25.53 |
| Acetonitrile | 50.93 | 41.67 |
| MIBK | 48.69 | 30.28 |

### f) Role of formic acid & DBU during Fmoc deprotection using piperidine: Aspartimide Impurity:

One of the major side reactions involved in Teduglutide synthesis is the formation of multiple aspartimide impurities, since there are five aspartic acid units in the sequence. Aspartimide impurity is formed at every deprotection stage due to the use of piperidine and other stronger bases.

### Mechanism for Aspartimide formation:

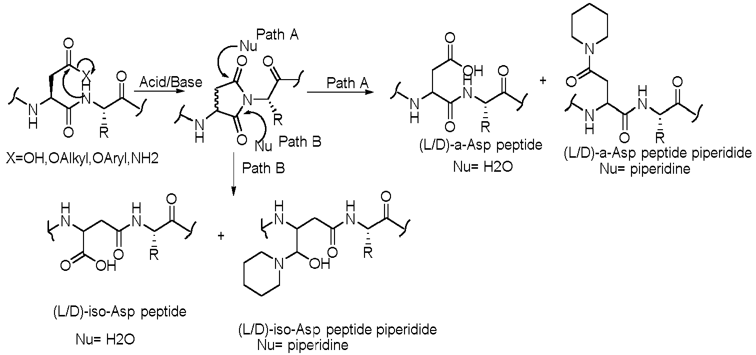

One of the aspects of the present invention/specification remedies the above problem in the process for preparation of Teduglutide wherein the Aspartimide impurity formation is minimized by incorporation of formic acid solution along with piperidine solution during Fmoc-deprotection. Piperidine abstracts proton from formic acid and forms piperidinium ion and thus minimizes aspartamide formation without affecting Fmoc-deprotection.

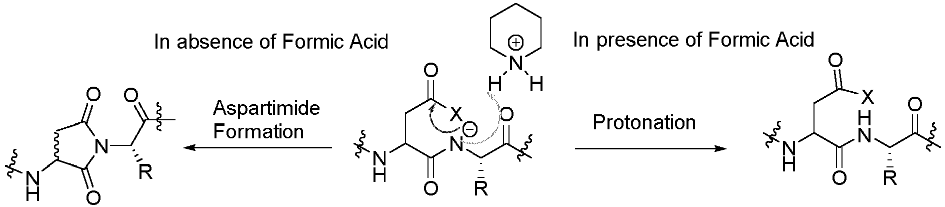

### Advantages of using DBU:

In certain stages of the process, due to the presence of hydrophobic amino acids shrinkage of the resin was observed during Fmoc-deprotection. This leads to incomplete deprotection of Fmoc-group and in-turn contributes to the formation of deletion impurities. To overcome the problem of incomplete deprotection Fmoc-group, 1- 3% DBU was incorporated in the reagent containing formic acid and piperidine. Efficient swelling of the resin is observed when DBU was incorporated which led to complete deprotection of Fmoc group.

### OBJECTIVE

The objective of the present invention/specification is to develop simple, robust, and commercially viable process for the preparation of Teduglutide of the **Formula I** optionally with the aid of inorganic salts.

### BRIEF DESCRIPTION OF DRAWINGS

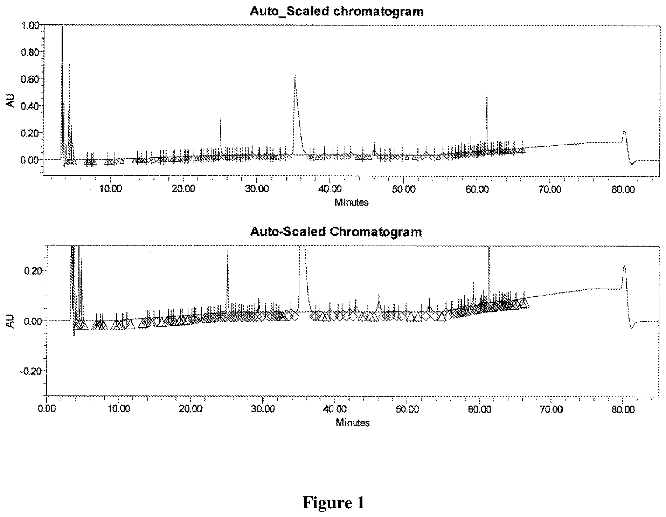

- Figure 1:: Illustrates HPLC chromatogram of Teduglutide.

### SUMMARY

One aspect of the present invention discloses a process for the preparation of a peptide with C-terminal Aspartic acid comprising the steps of:
a) Anchoring of first amino acid, aspartic acid to the Wang resin through its side chain carboxylic group;
b) Sequential coupling of side chain protected amino acids to prepare the desired peptide; in the presence of coupling agent and optionally in presence of chaotropic salt/s;
c) Crude peptide is obtained by removal of protective groups and cleavage of peptide from the resin;
d) Optionally purifying crude peptide.

In one aspect, the invention discloses a process for the preparation of Teduglutide comprising the steps of:
e) Anchoring of first amino acid, aspartic acid to the Wang resin through its side chain carboxylic group;
f) Sequential coupling of side chain protected amino acids to prepare Teduglutide, in the presence of coupling agent and optionally in presence of chaotropic salt/s;
g) Crude Teduglutide is obtained by removal of protective groups and cleavage of peptide from the resin;
h) Optionally purifying crude Teduglutide.

In another aspect, the invention discloses a process for the preparation of Teduglutide, comprising the steps of:
a) Anchoring of first amino acid, aspartic acid to the Wang resin through its side chain carboxylic group (Fmoc-Asp-OtBu);
b) Sequential coupling of side chain protected amino acids to prepare Teduglutide, in the presence of coupling agent and optionally in presence of chaotropic salt/s;
c) Usage of Piperidine: Formic acid: DBU mixture for the deprotection of Fmoc group;
d) Crude Teduglutide is obtained by removal of protective groups and cleavage of peptide from the resin;
e) Optionally purifying crude Teduglutide.

In another aspect, the invention discloses a process for the preparation of Teduglutide comprising the steps of:
a) Anchoring of first amino acid, aspartic acid to the Wang resin through its side chain carboxylic group (Fmoc-Asp-OtBu);
b) Sequential coupling of side chain protected amino acids to prepare Teduglutide, in the presence of coupling agent and Chaotropic salt/s;
c) Crude Teduglutide is obtained by removal of protective groups and cleavage of peptide from the resin using a cleavage cocktail;
d) Usage of free amino acids in the cleavage cocktail to reduce oxidated impurities.
e) Purification of crude Teduglutide;
   wherein free amino acids are selected from Methonine, Tryptophan and Histidine.

In another aspect, the invention discloses a process for the preparation of Teduglutide comprising the steps of:
a) Anchoring of first amino acid, aspartic acid to the Wang resin through its side chain carboxylic group (Fmoc-Asp-OtBu);
b) Sequential coupling of side chain protected amino acids to prepare Teduglutide, in the presence of coupling agent and Chaotropic salt/s;
c) Usage of Piperidine-Formic acid-DBU mixture for the deprotection of Fmoc group;
d) Crude Teduglutide is obtained by removal of protective groups and cleavage of peptide from the resin;
e) Usage of free amino acids and BHT in the cleavage cocktail to reduce oxidated impurities;
f) Purification of crude Teduglutide;
   wherein free amino acids are selected from Methonine, Tryptophan and Histidine.

In yet another aspect, the invention discloses a process for the cleavage of Teduglutide from the solid support using a cleavage solution, wherein the cleavage solution comprises of an antioxidant, amino acids and TFA cocktail.

The amino acids of the above aspect of the invention are selected from Methonine, Tryptophan and Histidine.

The antioxidant of the above aspect of the invention is selected from butylated hydroxytoluene (BHT).

In yet another aspect, the invention discloses, a process for the preparation of Teduglutide, wherein mono protected Histidine is used.

The protection group of the above aspect of the invention is *tert-*Butyloxycarbonyl (Boc).

The approach employed is solid phase peptide synthesis of Teduglutide by sequential approach and involves inorganic salts during coupling along with selective coupling agents and additives. The method offers completion of coupling and deprotection reactions and reduction in racemization and thereby control the isomeric impurities which are very close to the target molecule and in turn ease the purification process of the peptide.

### DETAILED DESCRIPTION OF THE INVENTION

The present invention relates to an efficient process for the preparation of Teduglutide by sequential coupling employing solid phase approach. It involves sequential coupling of protected amino acids to prepare Teduglutide, followed by removal of protective groups, cleavage of the peptide from solid support and purification of crude Teduglutide obtained.

The invention is represented by following examples. These examples are for illustration only and hence should not be construed as limitation of the scope of invention.

### ABBREVIATIONS:

| | |
|---|---|
| ACN | acetonitrile |
| °C | degree celsius |
| BHT | butylated hydroxytoluene |
| Boc | *tert-*Butyloxycarbonyl |
| COMU | (1-cyano-2-ethoxy-2-oxoethylidenaminooxy)dimethylamino-morpholino-carbenium hexafluorophosphate |
| CuCl₂ | cuprous chloride |
| DBU | 1,8-diazabicyclo[5.4.0]undec-7-ene |
| DEPBT | 3-(diethoxyphosphoryloxy)-1,2,3-benzotriazin-4(3H)-one |
| DIC | N,N'-diisopropylcarbodiimide |
| DIPEA | N,N-diisopropylethylamine |
| DMAP | 4-dimethylaminopyridine |
| DKP | diketopiperazine |
| DMF | dimethylformamide |
| DMS | dimethylsulfide |
| Eq. | equivalent |
| GLP-2 | glucagon-like peptide-2 |
| GVL | γ-valerolactone |
| h | hour |
| HBTU | *hexafluorophosphate* benzotriazole tetramethyl uronium |
| HCTU | O-(1H-6-chlorobenzotriazole-1-yl)-1,1,3,3-tetramethyluronium hexafluorophosphate |
| HOBt.H₂O | 1-hydroxybenzotriazole monohydrate |
| M | molar |
| Me | methyl |
| MDC | methylene dichloride |
| MIBK | methyl isobutyl ketone |
| MgCl₂ | magnesium chloride |
| NH₄I | ammonium iodide |
| NMM | N-methylmorpholine |
| NMP | N-methyl pyrrolidone |
| TFA | trifluoroacetic acid |
| TMP | tetramethylpiperidine |
| T₃P | propylphosphonic anhydride |
| v | volume |
| VTD | vacuum tray dryer |
| ZnCl₂ | zinc chloride |

The schematic description of the process is as shown as below:

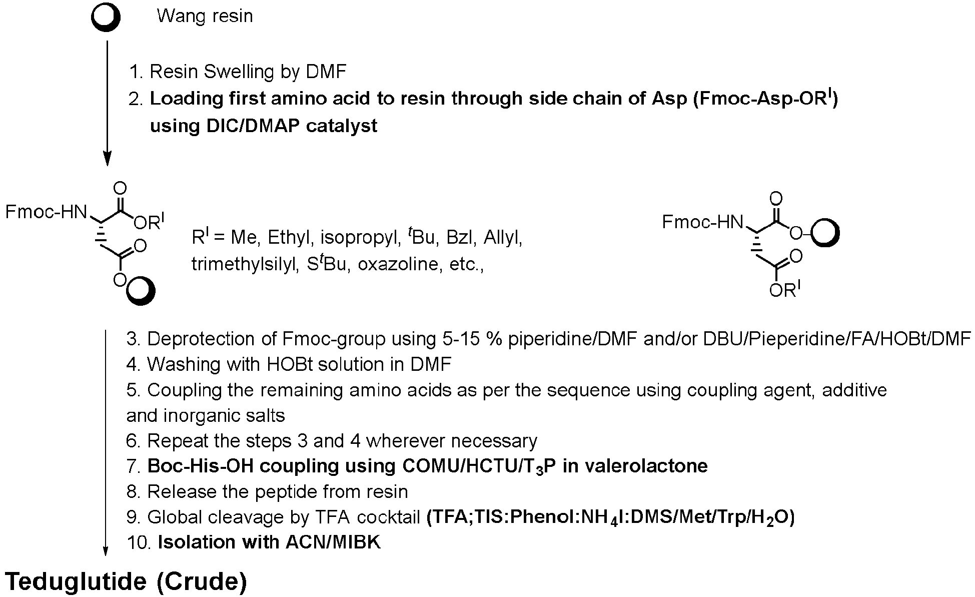

### Examples:

### Example 1: Synthesis of Teduglutide by anchoring aspartic acid (first amino acid) to Wang resin through α-carboxylic group of Asp.

The synthesis was performed by loading the C-terminal amino acid Fmoc-Asp(O*^{t}*Bu)-OH to Wang resin using DIPC and catalytic amount of DMAP in presence of MDC as solvent. The unreacted functional sites were capped using acetic anhydride and DIPEA. Fmoc deprotection was performed using 10-20 % piperidine solution in DMF twice for the period of 5 + 10 mins. Elongation of the peptide was carried out by sequentially addition of amino acids as per Teduglutide sequence. The resin with protected peptide obtained is as follows.

Boc-His(Trt)-Gly-Asp(OtBu)-Gly-Ser(tBu)-Phe-Ser(tBu)-Asp(OtBu)-Glu (OtBu)-Met-Asn(Trt)-Thr(tBu)-Ile-Leu-Asp(OtBu)-Asn(Trt)-Leu-Ala-Ala-Arg(Pbf)-Asp(OtBu)-Phe-Ile-Asn(Trt)-Trp(Boc)-Leu-Ile-Gln(Trt)-Thr(tBu)-Lys(Boc)-Ile-Thr (tBu)-Asp (OtBu) -Wang resin
Total cleavage was performed using TFA: TIS: Phenol as cocktail and the peptide was isolated using ether. Crude peptide with purity of 28.77 % was obtained.

### Example 2: Synthesis of Teduglutide by anchoring aspartic acid (first amino acid) to Wang resin through side chain carboxylic group of Asp.

### Step 1: Synthesis of Fmoc-Asp-O^{t}Bu³³- Wang resin

Wang resin (0.3-0.6 mmol/g, loading capacity) was loaded to the peptide synthesis vessel using 10 v of MDC, drained, added 7 v of MDC and swelling was performed for 1 h. The solvent was drained completely. Fmoc-Asp-O*^{t}*Bu (2.0 to 4.0 eq.) was dissolved in MDC and transferred to reaction vessel. DMAP (0.01 to 0.1 eq.) was dissolved in MDC and was added to the peptide synthesis vessel followed by DIPC (4.0 to 8.0 eq.). Esterification was performed for 1.0 to 3.0 h at room temperature. The reaction mass was drained and washed the amino acid loaded resin with MDC followed by DMF. Capping of the unreacted functional sites were carried out using acetic anhydride and DIPEA.

**Step 2:** Fmoc-deprotection of the loaded amino acid was performed by washing the resin with 5 to 15 % of piperidine in DMF for 5 and/or 10 minutes. The resin was washed with 0.01 to 0.1 M HOBt. H₂O in DMF (2 x 7 v) followed by DMF (5 x 7 v).

Fmoc-Thr (*^{t}*Bu)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 3:** Fmoc-deprotection of the loaded amino acid was performed by washing the resin with 5 to 15 % of piperidine in DMF for 5 and/or 10 minutes. The resin was washed with 0.01 to 0.1 M HOBt. H₂O in DMF (2 x 7 v) followed by DMF (5 x 7 v).

Fmoc-Ile-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 4:** Fmoc-deprotection of the loaded amino acid was performed by washing the resin with 5 to 15 % of piperidine in DMF for 5 and/or 10 minutes. The resin was washed with 0.01 to 0.1 M HOBt.H₂O in DMF (2 x 7 v) followed by DMF (5 x 7 v).

Fmoc-Lys(Boc)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 5**: Fmoc-deprotection of the loaded amino acid was performed by washing the resin with 5 to 15 % of piperidine in DMF for 5 and/or 10 minutes. The resin was washed with 0.01 to 0.1 M HOBt. H₂O in DMF (2 x 7 v) followed by DMF (5 x 7 v).

Fmoc-Thr(*^{t}*Bu)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 6:** Fmoc-deprotection of the loaded amino acid was performed by washing the resin with 5 to 20 % of piperidine in DMF for 5 and/or 10 minutes. The resin was washed with 0.01 to 0.1 M HOBt. H₂O in DMF (2 x 7 v) followed by DMF (5 x 7 v).

Fmoc-Gln(Trt)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 7:** Fmoc-deprotection of the loaded amino acid was performed by washing the resin with 5 to 20 % of piperidine in DMF for 5 and/or 10 minutes. The resin was washed with 0.01 to 0.1 M HOBt. H₂O in DMF (2 x 7 v) followed by DMF (5 x 7 v).

Fmoc-Ile-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 8:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid and 1 to 2 % of DBU and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Leu-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 9:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid and 1 to 2 % of DBU and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Trp(Boc)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 10:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid and 1 to 2 % of DBU and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Asn(Trt)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 11:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid and 1 to 2 % of DBU and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Ile-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C.

The reaction mass was drained and washed with DMF and/or NMP and/or MDC thrice. Completion of coupling was monitored by HPLC and/or Kaiser test.

**Step 12:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid and 1 to 2 % of DBU and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Phe-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 13:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid and 1 to 2 % of DBU and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc- Asp(O*^{t}*Bu)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 14:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid and 1 to 2 % of DBU and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Arg(Pbf)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 15:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid and 1 to 2 % of DBU and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Ala-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 16:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid and 1 to 2 % of DBU and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Ala-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 17:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid and 1 to 2 % of DBU and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Leu-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 18:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Asn(Trt)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 19:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Asp(O*^{t}*Bu)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 20:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Leu-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 21:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Ile-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 22:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Thr(*^{t}*Bu)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 23:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Asn(Trt)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 24:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Met-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 25:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Glu(O*^{t}*Bu)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 26:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Asp(O*^{t}*Bu)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 27:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Ser(*^{t}*Bu)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 28:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Phe-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 29:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Ser(*^{t}*Bu)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 30:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Gly-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 31:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc- Asp(O*^{t}*Bu)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 32:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Fmoc-Gly-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C. The reaction mass was drained and washed with DMF and/or NMP and/or MDC thrice. Completion of coupling was monitored by HPLC and/or Kaiser test.

**Step 33:** Fmoc-deprotection of the loaded amino acid was performed by washing the resin with 5 to 20 % of piperidine in DMF or 0.1 M to 1 M of formic acid with 5 to 20 % piperidine in DMF or 0.1 M to 1 M of formic acid, 1 to 3 % of DBU, and 5 to 20 % piperidine in DMF for 5 and/or 10 minutes. The resin was washed with 0.01 to 0.1 M HOBt. H₂O in DMF (2 x 7 v) followed by DMF (5 x 7 v).

Boc-His(Trt)-OH (2.0 to 4.0 eq.) was coupled using coupling agents such as HBTU, COMU, DEPBT and DIC, preferably HBTU (2 to 4.0 eq.) and additives such as oxymapure and HOBt.H₂O preferably HOBt.H₂O (2.0 to 4.0 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of DMF and/or NMP and/or MDC as solvent. The coupling reaction was performed for 1 to 2 h at 25 to 40 °C.

The reaction mass was drained and washed with DMF and/or NMP and/or MDC thrice. Completion of coupling was monitored by HPLC and/or Kaiser test.

The peptidyl resin obtained after the sequential addition of amino acids as per the sequence was washed twice with DMF, MDC, Methanol and MTBE. The resin was dried under vacuum in VTD and total cleavage was performed using TFA: TIS: Phenol in the ratio of 80:10:10. The cleavage was carried out at 20 to 30°C for 3 to 4 h under nitrogen atmosphere. The reaction mass was filtered and the filtrate containing the peptide and TFA cocktail was concentrated, and isolation of the peptide was carried out using solvent selected from MTBE, diisopropylether, MIBK, ACN and their mixtures thereof. The precipitated solid was centrifuged and/or filtered and washed with solvents selected from MTBE, diisopropylether, MIBK, ACN and any mixtures thereof, dried under vacuum in VTD and taken for purification in RP-HPLC. Purity of crude: 39.99 %.

### Example 3: Synthesis of crude peptide

The process remains same as described in example 2 till step 32. For the final amino acid i.e, His, coupling was carried out using Boc-His-OH (2.0 to 4.0 eq.) and coupling agents such as HCTU, COMU, DEPBT, T₃P and DIC, preferably COMU (2 to 4.0 eq.), DMAP (0.1 - 0.5 eq.) with base such as DIPEA, NMM, TMP preferably DIPEA and chaotropic salts such as MgCl₂, ZnCl₂, CuCl₂ preferably MgCl₂ (0.05 to 0.5 eq.) in presence of solvents selected from γ-valerolactone (GVL), DMF, NMP, MDC and any mixtures thereof. The coupling reaction was performed for 1 to 2 h at 25 to 40 °C. The reaction mass was drained and washed with DMF and/or NMP and/or MDC thrice.

The peptidyl resin obtained after the sequential addition of amino acids as per the sequence was washed twice with DMF, MDC, Methanol and MTBE. The resin was dried under vacuum in VTD and total cleavage was performed using TFA: TIS: Phenol: NH₄I: DMS in the ratio of 80: 7.5:7.5:5:5. The cleavage was carried out at 20 to 30 °C for 3 to 4 h under nitrogen atmosphere. The reaction mass was filtered and the filtrate containing the peptide and TFA cocktail was concentrated, and isolation of the peptide was carried out using solvents selected from MTBE, diisopropylether, MIBK, ACN and any mixtures thereof. The precipitated solid was centrifuged and/or filtered and washed with solvents selected from MTBE, diisopropylether, MIBK, ACN and any mixtures thereof and dried under vacuum in VTD and taken for purification in RP-HPLC. Purity of crude: 46.69 %

### Example 4: Cleavage using TFA: TIS: Phenol: NH₄I: DMS (80: 7.5:7.5:5:5)

The process is same as described in example 2 till step 32. Coupling of 33^{rd} amino acid i.e, His was carried out as per example 3. The dried peptidyl resin (5 g) was taken and treated with 10 to 15 v of cocktail containing TFA: TIS: Phenol: NH₄I: DMS in the ratio of 80: 7.5:7.5:5:5. The cleavage was carried out at 20 to 30 °C for 3 to 4 h under nitrogen atmosphere. The reaction mass was filtered and the filtrate containing the peptide and TFA cocktail was concentrated, and isolation of the peptide was carried out using solvents selected from MTBE, diisopropylether, MIBK, ACN and any mixtures thereof. The precipitated solid was centrifuged and/or filtered and washed with solvents selected from MTBE, diisopropylether, MIBK, ACN and any mixtures thereof, dried under vacuum in VTD and taken for purification in RP-HPLC. Purity of crude: 47.7 %, API content: 0.64 g

### Example 5: Cleavage using TFA: TIS: Phenol: NH₄I: DMS: Met (75: 5: 5: 5: 5: 5)

The process is same as described in example 2. Coupling of 33^{rd} amino acid i.e, His was carried out as per example 3. The dried peptidyl resin (5 g) was taken and treated with 10 to 15 v of cocktail containing TFA: TIS: Phenol: NH₄I: DMS: Met in the ratio of 75: 5: 5: 5: 5: 5. The cleavage was carried out at 20 to 30 °C for 3 to 4 h under nitrogen atmosphere. The reaction mass was filtered and the filtrate containing the peptide and TFA cocktail was concentrated, and isolation of the peptide was carried out using solvents selected from MTBE, diisopropylether, MIBK, ACN and any mixtures thereof. The precipitated solid was centrifuged and/or filtered and washed with solvents selected from MTBE, diisopropylether, MIBK, ACN and any mixtures thereof, dried under vacuum in VTD and taken for purification in RP-HPLC. Purity of crude: 48.94 %, API content: 0.74 g

### Example 6: Cleavage using TFA: TIS: Phenol: NH₄I: DMS: Trp (75: 5: 5: 5: 5: 5)

The process is same as described in example 2. Coupling of 33^{rd} amino acid i.e, His was carried out as per example 3. The dried peptidyl resin (5 g) was taken and treated with 10 to 15 v of cocktail containing TFA: TIS: Phenol: NH₄I: DMS: Trp in the ratio of 75: 5: 5: 5: 5: 5. The cleavage was carried out at 20 to 30 °C for 3 to 4 h under nitrogen atmosphere. The reaction mass was filtered and the filtrate containing the peptide and TFA cocktail was concentrated, and isolation of the peptide was carried out using solvents selected from MTBE, diisopropylether, MIBK, ACN and any mixtures thereof. The precipitated solid was centrifuged and/or filtered and washed with Solvents selected from MTBE, diisopropylether, MIBK, ACN and any mixtures thereof, dried under vacuum in VTD and taken for purification in RP-HPLC. Purity of crude: 43.85 %, API content: 0.76 g

### Example 7: Cleavage using TFA: TIS: Phenol: NH₄I: DMS: Trp: Met (75: 5: 5: 5: 5: 2.5: 2.5)

The process is same as described in example 2. Coupling of 33^{rd} amino acid i.e, His was carried out as per example 3. The dried peptidyl resin (5 g) was taken and treated with 10 to 15 v of cocktail containing TFA: TIS: Phenol: NH₄I: DMS: Trp: Met in the ratio of 75: 5: 5: 5: 5: 2.5: 2.5. The cleavage was carried out at 20 to 30 °C for 3 to 4 h under nitrogen atmosphere. The reaction mass was filtered and the filtrate containing the peptide and TFA cocktail was concentrated, and isolation of the peptide was carried out using solvents selected from MTBE, diisopropylether, MIBK, ACN and any mixtures thereof. The precipitated solid was centrifuged and/or filtered and washed with Solvents selected from MTBE, diisopropylether, MIBK, ACN and any mixtures thereof, dried under vacuum in VTD and taken for purification in RP-HPLC. Purity of crude: 46.06 %, API content: 0.75 g

### Example 8: Cleavage using TFA: TIS: Phenol: NH₄I: DMS: Trp: Met: Water (75: 4: 5: 5: 5: 2.5: 2.5: 1)

The process is same as described in example 2. Coupling of 33^{rd} amino acid i.e, His was carried out as per example 3. The dried peptidyl resin (5 g) was taken and treated with 10 to 15 v of cocktail containing TFA: TIS: Phenol: NH₄I: DMS: Trp: Met : Water in the ratio of 75: 4: 5: 5: 5: 2.5: 2.5: 1. The cleavage was carried out at 20 to 30 °C for 3 to 4 h under nitrogen atmosphere. The reaction mass was filtered and the filtrate containing the peptide and TFA cocktail was concentrated, and isolation of the peptide was carried out using solvents selected from MTBE, diisopropylether, MIBK, ACN and any mixtures thereof. The precipitated solid was centrifuged and/or filtered and washed with Solvents selected from MTBE, diisopropylether, MIBK, ACN and any mixtures thereof, dried under vacuum in VTD and taken for purification in RP-HPLC. Purity of crude: 48.09 %, API content: 0.72 g.

### Example 9: Cleavage using TFA: TIS: Phenol: NH₄I: DMS: Trp: Met: BHT (74: 7.5: 7.5: 2.5: 2.5: 2.5: 2.5: 1)

The process is same as described in example 2. Coupling of 33^{rd} amino acid i.e, His was carried out as per example 3. The dried peptidyl resin (200 g) was taken and treated with 10 to 15 v of cocktail containing TFA: TIS: Phenol: NH₄I: DMS: Trp: Met: BHT in the ratio of 74: 7.5: 7.5: 2.5: 2.5: 2.5: 2.5: 1. The cleavage was carried out at 20 to 30 °C for 3 to 4 h under nitrogen atmosphere. The reaction mass was filtered and the filtrate containing the peptide and TFA cocktail was concentrated, and isolation of the peptide was carried out using solvents selected from MTBE, diisopropylether, MIBK, ACN and any mixtures thereof. The precipitated solid was centrifuged and/or filtered and washed with Solvents selected from MTBE, diisopropylether, MIBK, ACN and any mixtures thereof, dried under vacuum in VTD and taken for purification in RP-HPLC. Purity of crude: 52.54 %, API content: 36.40 g.

## Claims

1. A process for the preparation of Teduglutide comprising the steps of:
a) anchoring the first amino acid, aspartic acid, to the Wang resin through its side chain carboxylic group;
b) sequentially coupling side chain protected amino acids to prepare Teduglutide, in the presence of a coupling agent; and
c) obtaining crude Teduglutide by removing protective groups and cleaving the peptide from the resin.

2. The process according to claim 1, wherein step (b) is carried out in the presence of a chaotropic salt.

3. The process according to claim 1 or 2, further comprising
d) purifying crude Teduglutide.

4. The process according to any of claims 1 to 3, wherein for anchoring the first amino acid, aspartic acid, to the Wang resin, Fmoc-Asp-OtBu is used.

5. The process according to any of claims 1 to 4, comprising the steps of:
a) anchoring of first amino acid, aspartic acid to the Wang resin through its side chain carboxylic group (Fmoc-Asp-OtBu);
b) coupling sequentially of side chain protected amino acids to prepare Teduglutide, in the presence of coupling agent and optionally in presence of a chaotropic salt;
c) using a Piperidine: Formic acid: DBU mixture for the deprotection of Fmoc group;
d) obtaining crude Teduglutide by removal of protective groups and cleavage of peptide from the resin;
e) optionally purifying crude Teduglutide.

6. The process of any of the preceding claims, wherein the DBU mixture comprises 1-3% DBU.

7. The process of any of the preceding claims, wherein cleaving the peptide from the resin comprises using a cleavage cocktail that comprises free amino acids selected from Methonine, Tryptophan and Histidine.

8. The process of any of the preceding claims, wherein cleaving the peptide from the resin further comprises using the antioxidant butylated hydroxytoluene (BHT).

9. The process of any of the preceding claims, wherein cleaving the peptide from the resin comprises using a cleavage solution comprising an antioxidant, free amino acids and TFA.

10. The process of any of the preceding claims, wherein the side chain protected amino acids in step (b) comprise side chain protected Histidine as mono protected Histidine.

11. The process of claim 10, wherein the protection group of the mono protected Histidine is *tert-*Butyloxycarbonyl (Boc).

12. The use of a Wang resin in the synthesis of Teduglutide, wherein the use comprises anchoring aspartic acid to a Wang resin through its side chain carboxylic group.

13. The use of claim 12, wherein the formation of diketopiperazine is minimized.

14. The use of a Wang resin according to claim 12 or 13, further comprising the use of free Methonine, free Tryptophan and/or free Histidine in the cleavage cocktail in the synthesis of Teduglutide.

15. The use of claim 14, wherein the formation of oxidative impurities is reduced.

## Patentansprüche

1. Verfahren zur Herstellung von Teduglutid, aufweisend die folgenden Schritte:
a) die erste Aminosäure, Asparaginsäure, über ihre Seitenketten-Carboxylgruppe an dem Wang-Harz verankern;
b) sequentiell seitenkettengeschützte Aminosäuren zur Herstellung von Teduglutid in Gegenwart eines Kupplungsmittels ankoppeln; und
c) rohes Teduglutid durch Entfernen der Schutzgruppen und Abspalten des Peptids vom Harz gewinnen.

2. Verfahren nach Anspruch 1, wobei Schritt (b) in Gegenwart eines chaotropen Salzes durchgeführt wird.

3. Verfahren nach Anspruch 1 oder 2, das ferner aufweist
d) das rohe Teduglutid reinigen.

4. Verfahren nach einem der Ansprüche 1 bis 3, wobei zum Verankern der ersten Aminosäure, Asparaginsäure, an das Wang-Harz Fmoc-Asp-OtBu verwendet wird.

5. Verfahren nach einem der Ansprüche 1 bis 4, aufweisend die folgenden Schritte:
a) der ersten Aminosäure, Asparaginsäure, an das Wang-Harz über ihre Seitenketten-Carboxylgruppe (Fmoc-Asp-OtBu) verankern;
b) sequentiell seitenkettengeschützte Aminosäuren zur Herstellung von Teduglutid in Gegenwart eines Kupplungsmittels und gegebenenfalls in Gegenwart eines chaotropen Salzes ankoppeln;
c) eine Mischung aus Piperidin, Ameisensäure und DBU zum Entschützen der Fmoc-Gruppe verwenden;
d) rohes Teduglutid durch Entfernen der Schutzgruppen und Abspaltung des Peptids vom Harz gewinnen;
e) optional das rohe Teduglutid reinigen.

6. Verfahren nach einem der vorangehenden Ansprüche, wobei die DBU-Mischung 1-3 % DBU aufweist.

7. Verfahren nach einem der vorangehenden Ansprüche, wobei das Abspalten des Peptids vom Harz die Verwendung eines Spaltcocktails aufweist, der freie Aminosäuren aufweist, die aus Methionin, Tryptophan und Histidin ausgewählt sind.

8. Verfahren nach einem der vorangehenden Ansprüche, wobei das Abspalten des Peptids vom Harz weiterhin die Verwendung des Antioxidans Butylhydroxytoluol (BHT) aufweist.

9. Verfahren nach einem der vorangehenden Ansprüche, wobei das Abspalten des Peptids vom Harz die Verwendung einer Spaltlösung aufweist, die ein Antioxidans, freie Aminosäuren und TFA aufweist.

10. Verfahren nach einem der vorangehenden Ansprüche, wobei die seitenkettengeschützten Aminosäuren in Schritt (b) seitenkettengeschütztes Histidin als monogeschütztes Histidin aufweisen.

11. Verfahren nach Anspruch 10, wobei die Schutzgruppe des monoschutzten Histidins tert-Butyloxycarbonyl (Boc) ist.

12. Verwendung eines Wang-Harzes bei der Synthese von Teduglutid, wobei die Verwendung die Verankerung von Asparaginsäure an ein Wang-Harz über deren Carboxylgruppe in der Seitenkette aufweist.

13. Verwendung nach Anspruch 12, wobei die Bildung von Diketopiperazin minimiert wird.

14. Verwendung eines Wang-Harzes gemäß Anspruch 12 oder 13, weiterhin aufweisend die Verwendung von freiem Methionin, freiem Tryptophan und/oder freiem Histidin im Spaltcocktail bei der Synthese von Teduglutid.

15. Verwendung nach Anspruch 14, wobei die Bildung oxidativer Verunreinigungen verringert wird.

## Revendications

1. Processus de préparation du téduglutide comprenant les étapes consistant à :
a) ancrer le premier acide aminé, l'acide aspartique, à la résine de Wang par l'intermédiaire de son groupe carboxylique porté par sa chaîne latérale ;
b) coupler de manière séquentielle les acides aminés protégés sur la chaîne latérale pour préparer le téduglutide, en présence d'un agent de couplage ; et
c) obtenir du téduglutide brut par élimination des groupes protecteurs et clivage du peptide à partir de la résine.

2. Processus selon la revendication 1, dans lequel l'étape (b) est réalisée en présence d'un sel chaotropique.

3. Processus selon la revendication 1 ou 2, comprenant en outre :
d) la purification du téduglutide brut.

4. Processus selon l'une quelconque des revendications 1 à 3, dans lequel pour ancrer le premier acide aminé, l'acide aspartique, à la résine de Wang, on utilise du Fmoc-Asp-OtBu.

5. Processus selon l'une quelconque des revendications 1 à 4, comprenant les étapes consistant à :
a) ancrer le premier acide aminé, l'acide aspartique, à la résine de Wang par l'intermédiaire de son groupe carboxylique porté par sa chaîne latérale (Fmoc-Asp-OtBu) ;
b) coupler de manière séquentielle les acides aminés protégés sur la chaîne latérale pour préparer le téduglutide, en présence d'un agent de couplage et optionnellement en présence d'un sel chaotropique ;
c) utiliser un mélange Pipéridine : Acide formique : DBU pour la déprotection du groupe Fmoc ;
d) obtenir du téduglutide brut par élimination des groupes protecteurs et clivage du peptide de la résine ;
e) optionnellement, purifier le téduglutide brut.

6. Processus selon l'une quelconque des revendications précédentes, dans lequel le mélange DBU comprend de 1 à 3 % de DBU.

7. Processus selon l'une quelconque des revendications précédentes, dans lequel le clivage du peptide de la résine comprend l'utilisation d'un cocktail de clivage qui comprend des acides aminés libres sélectionnés parmi la méthionine, le tryptophane et l'histidine.

8. Processus selon l'une quelconque des revendications précédentes, dans lequel le clivage du peptide à partir de la résine comprend en outre l'utilisation de l'antioxydant hydroxytoluène butylé (BHT).

9. Processus selon l'une quelconque des revendications précédentes, dans lequel le clivage du peptide de la résine comprend l'utilisation d'une solution de clivage comprenant un antioxydant, des acides aminés libres et du TFA.

10. Processus selon l'une quelconque des revendications précédentes, dans lequel les acides aminés protégés sur la chaîne latérale de l'étape (b) comprennent de l'histidine protégée sur la chaîne latérale sous forme d'histidine mono-protégée.

11. Processus selon la revendication 10, dans lequel le groupe protecteur de l'histidine mono-protégée est le tert-butyloxycarbonyle (Boc).

12. Utilisation d'une résine de Wang dans la synthèse du téduglutide, dans laquelle l'utilisation comprend l'ancrage d'acide aspartique à une résine de Wang par l'intermédiaire de son groupe carboxylique porté par sa chaîne latérale.

13. Utilisation selon la revendication 12, dans laquelle la formation de dicétopipérazine est minimisée.

14. Utilisation d'une résine de Wang selon la revendication 12 ou 13, comprenant en outre l'utilisation de méthionine libre, de tryptophane libre et/ou d'histidine libre dans le cocktail de clivage dans la synthèse du téduglutide.

15. Utilisation selon la revendication 14, dans laquelle la formation d'impuretés oxydatives est réduite.
